# EUROPEAN PATENT SPECIFICATION

(11) **EP 4 233 983 B1**
(45) Date of publication and mention of the grant of the patent: **21.10.2026**
(21) Application number: 23176620.5
(22) Date of filing: 03.01.2019
(51) Int. Cl.: A61M 39/10, A61M 5/36, A61M 39/20

(54) **FLOW CONTROL PLUG SECUREMENT**
FLUSSSTEUERUNGSSTOPFEN-SICHERUNG
FIXATION DU BOUCHON DE CONTRÔLE DE DÉBIT

(30) Priority: 14.02.2018 US 201815896257
(43) Date of publication of application: 30.08.2023
(62) Document number(s) of the earlier application(s) in accordance with Art. 76 EPC: 19702140.5 / 3 752 240
(73) Proprietor: Becton, Dickinson and Company, Franklin Lakes, NJ 07417-1880 (US)
(72) Inventor: KARTHIKEYAN, Sakthivel, 641025 Coimbatore (IN); SRINIVASAN, Sudarsan, New Jersey, 08902 (US)
(74) Representative: dompatent

(56) References cited:
- WO-A1-2012/009457
- US-A- 5 269 771
- US-A1- 2017 239 443
- US-B2- 8 377 040

## Description

### BACKGROUND OF THE INVENTION

Infusion therapy is one of the most common health care procedures. Hospitalized, home care, and other patients receive fluids, pharmaceuticals, and blood products via a vascular access device inserted into the vascular system. Infusion therapy may be used to treat an infection, provide anesthesia or analgesia, provide nutritional support, treat cancerous growths, maintain blood pressure and heart rhythm, or many other clinically significant uses.

Infusion therapy is facilitated by a vascular access device. The vascular access device may access a patient's peripheral or central vasculature. The vascular access device may be indwelling for short term (days), moderate term (weeks), or long term (months to years). The vascular access device may be used for continuous infusion therapy or for intermittent therapy.

A common vascular access device is a catheter that is inserted into a patient's vein. The catheter length may vary from a few centimeters for peripheral access to many centimeters for central access. The catheter may be inserted transcutaneously or may be surgically implanted beneath the patient's skin. The catheter may have a single lumen or multiple lumens for infusion of many fluids simultaneously.

One example of a catheter system that includes a catheter is the BD NEXIVA^{™} Closed IV (intravenous) Catheter System, by Becton, Dickinson and Company. This system includes an over-the-needle, peripheral intravascular catheter, integrated extension tubing, a Y-adapter, a slide clamp, a flow control plug, a Luer access port, and a passive needle-shielding mechanism.

The design of the BD NEXIVA^{™} IV Catheter System can be described as a closed system since it protects clinicians or operators from blood exposure during the catheter insertion procedure. Since the needle is withdrawn through a septum that seals, after the needle has been removed and both ports of the Y-adapter are closed, blood is contained within the NEXIVA^{™} device during catheter insertion. The pressure exerted on the needle as it passes through the septum wipes blood from the needle, further reducing potential blood exposure. The clamp on the integrated extension tubing is provided to eliminate blood exposure when the flow control plug is replaced with another vascular access device such as an infusion set connection or a Luer access port.

A current procedure of initiating the use of a catheter system such as the BD NEXIVA^{™} Closed IV Catheter System is as follows. A device operator will insert the needle into the vasculature of a patient and wait for flashback of blood to travel into the device to confirm that the needle is properly located within the vasculature of the patient. The blood travels into and along the catheter of the device because a flow control plug permits air to escape the device as blood enters the device. In some instances, after an operator confirms proper placement, the operator may clamp the catheter system to halt the progression of blood through the catheter system, remove the flow control plug, replace the flow control plug with another vascular access device such as an infusion set connection or a Luer access port, unclamp the catheter system, flush the blood from the catheter system back into the vasculature of the patient, and re-clamp the catheter system.

Further background art for the present invention is described in US 2017/239443 A1, US 5 269 771 A and D3 US 8 377 040 B2.

### BRIEF SUMMARY OF THE INVENTION

The present invention is defined in the independent claims1 and 9.

The present disclosure relates generally to a flow control plug and related devices, systems, and methods. In some embodiments, the flow control plug may be secured to a luer adapter of a catheter system in a secure manner, which may prevent accidental uncoupling of the flow control plug from the luer adapter prior to use. If the flow control plug prematurely uncouples from the luer adapter, contamination of the flow control plug may result, making the flow control plug unable to be used. In some embodiments, the flow control plug may also be secured to the luer adapter in a fluid-tight manner, which may importantly prevent leakage of fluid from the catheter system. In some embodiments, the flow control plug may also include one or more features that facilitate attachment of the flow control plug to the luer adapter during assembly or prior to use, including, for example, grip elements and/or flexible arms. In some embodiments, the flow control plug may include one or more features that reduce an axial force and torque necessary to make a connection between the flow control plug and the luer adapter.

As used in the present disclosure, the term "distal" refers to a portion of the IV catheter system or component thereof that is farther from a user, and the term "proximal" refers to a portion of the IV catheter system or component thereof that is closer to the user. As used in the present disclosure, the term "user" may refer to a clinician, doctor, nurse, or any other care provider and may include support personnel.

In some embodiments, the catheter system may include one or more of the following: a catheter adapter, a catheter extending distally from the catheter adapter, a needle extending through the catheter and beyond a distal tip of the catheter when the needle is in an insertion position, the luer adapter, extension tubing, and the flow control plug. In some embodiments, the catheter adapter may include a side port, which may be coupled to the extension tubing. In some embodiments, the catheter adapter may include a septum, which may be disposed in a needle channel through which the needle travels in response to being retracted. In some embodiments, a fluid pathway of the catheter adapter may include one or more of the following: a lumen of the catheter adapter distal to the septum, the side port, the extension tubing, and the luer adapter. In some embodiments, the luer adapter may be connectable to another vascular access device, such as blood withdrawal and/or infusion means.

In some embodiments, the catheter system may include an intravenous (IV) catheter system. In some embodiments, the catheter system may include an integrated or a closed catheter system, such as, for example, the BD NEXIVA^{™} Closed IV Catheter System, the BD NEXIVA^{™} DIFFUSICS^{™} Closed IV Catheter System, the BD SAF-T-INTIMA^{™} Closed IV Catheter System, or the Becton Dickinson PEGASUS^{™} Safety Closed IV Catheter System.

In some embodiments, the luer adapter may include a single port adapter, a dual port adapter, a Y-adapter, or another suitable type of luer adapter. The luer adapter includes an outer surface, which includes a recess. The luer adapter is coupled to a catheter adapter via extension tubing. The catheter system includes a flow control plug, which includes a proximal end and a distal end. The proximal end of the flow control plug includes a filter element, which is permeable to air and not permeable to blood. The distal end of the flow control plug includes a cylinder and a taper-shaped luer tip spaced apart from the cylinder.

An inner surface of the cylinder includes a protrusion engaged in a snap-fit with the recess, which may secure the flow control plug to the luer adapter.
In some embodiments, the outer surface of the luer adapter may include threading. In some embodiments, the distal end of the flow control plug may include corresponding threading, which may be mated with the threading to secure the flow control plug to the luer adapter.

The cylinder includes multiple slots, which form multiple arms of the cylinder. The arms are disposed between the slots. In some embodiments, the slots may extend through the cylinder. An inner surface of one of the arms includes the protrusion. In some embodiments, multiple of the arms may each include another protrusion, which may engage in a snap-fit with the recess or with another recess in the outer surface of the luer adapter. For example, the slots may include a first slot and a second slot, and the arms may include a first arm and a second arm. In some embodiments, the first slot and the second slot may be disposed between the first arm and the second arm. In some embodiments, an inner surface of the first arm may include the protrusion. In some embodiments, an inner surface of the second arm may include another protrusion. In some embodiments, the other protrusion may be engaged in a snap-fit with the recess or with another recess of the outer surface of the luer adapter.

In some embodiments, the distal end may include a stepped surface. In some embodiments, the distal end may extend outwardly from the proximal end to form the stepped surface. In some embodiments, the slots may extend through the stepped surface. In some embodiments, the stepped surface may be annular.

In some embodiments, an outer surface of the cylinder may include one or more first grip elements. For example, outer surfaces of one or more of the arms may include one or more first grip elements. In some embodiments, the first grip elements may include ribs, which may be oriented longitudinally with respect to the flow control plug. In some embodiments, an outer surface of the proximal end may include one or more second grip elements. In some embodiments, the second grip elements may include ribs, which may be oriented longitudinally with respect to the flow control plug. In some embodiments, the cylinder may include a first number of the first grip elements and/or the outer surface of the proximal end may include a second number of the second grip elements. In some embodiments, the second number may be greater than the first number. In some embodiments, a width of each of the first grip elements may be greater than a width of each of the second grip elements.

The method of securing the flow control plug to the catheter system for shipment according to the invention includes providing the catheter system and coupling the flow control plug to the luer adapter of the catheter system before shipping the catheter system to a destination. Coupling the flow control plug to the luer adapter of the catheter system before shipping the catheter system to the destination includes engaging the recess with the protrusion in a snap-fit or mating the threading to the corresponding threading.

It is to be understood that both the foregoing general description and the following detailed description are exemplary and explanatory and are not restrictive of the invention, as aspected.

### BRIEF DESCRIPTION OF THE SEVERAL VIEWS OF THE DRAWINGS

Example embodiments will be described and explained with additional specificity and detail through the use of the accompanying drawings in which:
Figure 1 is an upper perspective view of an example catheter system, according to some embodiments;
Figure 2A is an upper perspective view of an example threaded luer adapter, according to some embodiments;
Figure 2B is a top view of the threaded luer adapter of Figure 2A, according to some embodiments;
Figure 2C is a side view of the threaded luer adapter of Figure 2A, according to some embodiments;
Figure 2D is a cross-sectional view of the threaded luer adapter of Figure 2A, along the line 2D-2D of Figure 2C, according to some embodiments;
Figure 2E is a proximal end view of the threaded luer adapter of Figure 2A, according to some embodiments;
Figure 3A is an upper perspective view of an example threaded flow control plug, according to some embodiments;
Figure 3B is a top view the threaded flow control plug of Figure 3A, according to some embodiments;
Figure 3C is a cross-sectional view of the threaded flow control plug of Figure 3A, along the line 3C-3C of Figure 3B, according to some embodiments;
Figure 4A is an upper perspective view of the threaded luer adapter of Figure 2A coupled to the threaded flow control plug of Figure 3A, according to some embodiments;
Figure 4B is a top view of the threaded luer adapter of Figure 2A coupled to the threaded flow control plug of Figure 3A, according to some embodiments;
Figure 4C is a cross-sectional view, along the line 4C-4C of Figure 4B, of the threaded luer adapter of Figure 2A coupled to the threaded flow control plug of Figure 3A, according to some embodiments;
Figure 4D is a side view of the threaded luer adapter of Figure 2A coupled to the threaded flow control plug of Figure 3A, according to some embodiments;
Figure 5A is an upper perspective view of another flow control plug, according to some embodiments;
Figure 5B is a top view of the flow control plug of Figure 5A, according to some embodiments;
Figure 5C is a cross-sectional view of the flow control plug of Figure 5A, along the line 5C-5C of Figure 5B, according to some embodiments;
Figure 6A is an upper perspective view of the flow control plug of Figure 5A coupled with another luer adapter, according to some embodiments;
Figure 6B is a top view of the flow control plug of Figure 5A coupled with the other luer adapter of Figure 6A, according to some embodiments;
Figure 6C is a cross-sectional view, along the line 6C-6C of Figure 6B, of the flow control plug of Figure 5A coupled with the other luer adapter of Figure 6A, according to some embodiments;
Figure 6D is a side view of the flow control plug of Figure 5A coupled with the other luer adapter of Figure 6A, according to some embodiments;
Figure 7A is an upper perspective view another example flow control plug, according to some embodiments;
Figure 7B is a top view of the flow control plug of Figure 7A, according to some embodiments;
Figure 7C is a cross-sectional view of the flow control plug of Figure 7A, along line 7C-7C of Figure 7B, according to some embodiments;
Figure 7D is a side view of the flow control plug of Figure 7A, according to some embodiments;
Figure 8A is an upper perspective view of the flow control plug of Figure 7A coupled with the other luer adapter of Figure 6A, according to some embodiments;
Figure 8B is a top view of the flow control plug of Figure 7A coupled with the other luer adapter of Figure 6A, according to some embodiments;
Figure 8C is a cross-sectional view, along the line 8C-8C of Figure 8B, of the flow control plug of Figure 7A coupled with the other luer adapter of Figure 6A, according to some embodiments; and
Figure 8D is a side view of the flow control plug of Figure 7A coupled with the other luer adapter of Figure 6A, according to some embodiments.

### DETAILED DESCRIPTION OF THE INVENTION

The presently preferred embodiments of the present invention will be best understood by reference to the drawings, wherein like reference numbers indicate identical or functionally similar elements. It will be readily understood that the components of the present invention, as generally described and illustrated in the figures herein, could be arranged and designed in a wide variety of different configurations. Thus, the following more detailed description, as represented in the figures, is not intended to limit the scope of the invention as aspected, but is merely representative of presently preferred embodiments of the invention.

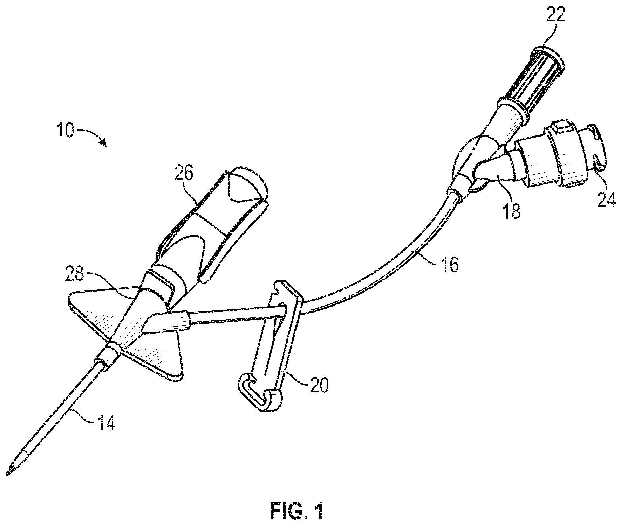

Referring now to FIG. 1, a catheter system 10, such as, for example, the BD NEXIVA^{™} Closed IV (intravenous) Catheter System, by Becton, Dickinson and Company, is used to communicate fluid with the vascular system of a patient. An example of the catheter system 10, as shown in FIG. 1, includes an introducer needle 12; an over-the-needle, peripheral intravascular catheter 14 made from polyurethane; an integrated extension tubing 16 with a Y-adapter 18 and slide clamp 20; a flow control plug 22; a Luer access port 24; a passive needle-shielding mechanism 26; and a catheter adapter 28 from which the catheter 14 extends. In some embodiments, any adapter used to connect two or more vascular access devices may be used in place of the Y-adapter 18.

The catheter system 10 is a closed system since it protects a user from blood exposure during the catheter 14 insertion procedure. Since the needle 12 is withdrawn through a septum that seals after the needle 12 has been removed and both ports of the Y-adapter 18 are closed, blood is contained within the catheter system 10 during catheter 14 insertion. The pressure exerted on the needle 12 as it passes through the septum wipes blood from the needle 12, further reducing potential blood exposure. The slide clamp 20 on the integrated extension tubing 16 is provided to eliminate blood exposure when the flow control plug 22 is replaced with another vascular access device such as an infusion set connection or another Luer access port 24.

As mentioned above, a current procedure of initiating the use of the extravascular system 10 is as follows. The user may insert the needle 12 into the vasculature of a patient and wait for flashback of blood to travel into the catheter system 10 to confirm that the needle 12 is properly located within the vasculature of the patient. The blood travels into and along the catheter 14 in the space between the needle 12 and the catheter 14. This occurs because a flow control plug 22 permits air to escape the catheter system 10 as blood enters the catheter system 10. After an operator confirms proper placement, and after adequate venting of the catheter system 10 has occurred, the user may clamp extension tubing 16 to halt the progression of blood through the catheter 14, remove the flow control plug 22, replace the flow control plug 22 with another vascular access device such as an infusion set connection or a Luer access port similar or identical to Luer access port 24. In some instances, the user may then unclamp the extension tubing 16, flush the blood from the catheter 14 back into the vasculature of the patient, and re-clamp the extension tubing 16. Alternate flow control plugs, luer adapters, and venting procedures are desired and will be discussed with reference to the figures following FIG. 1.

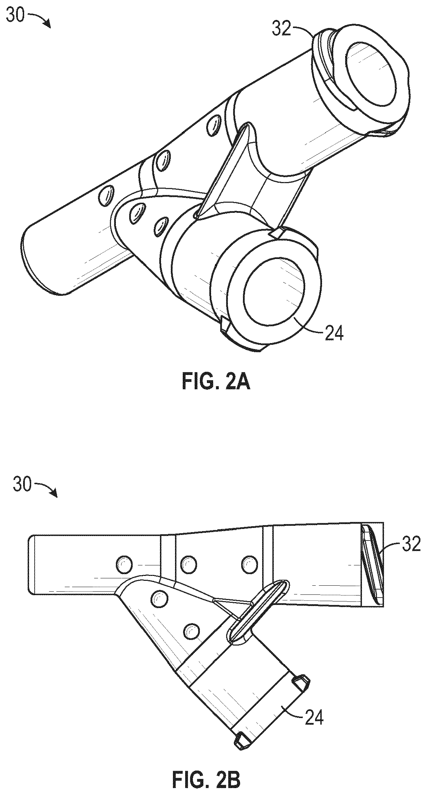

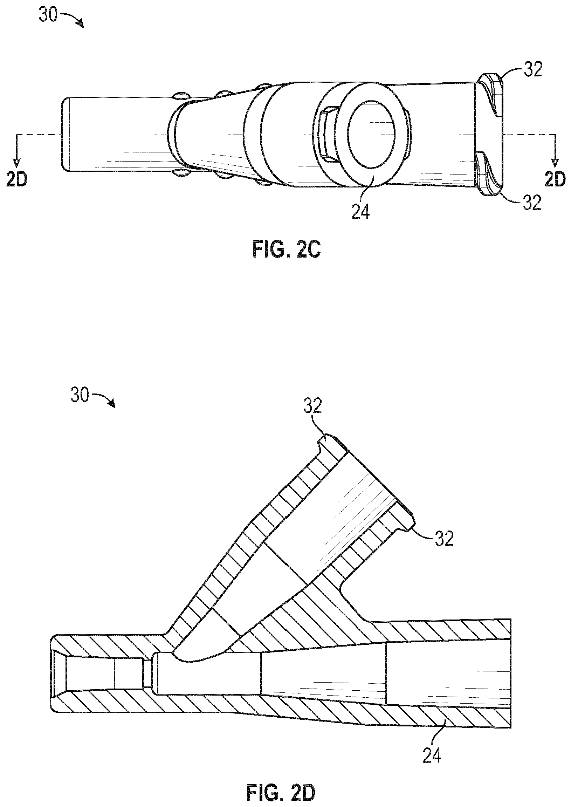

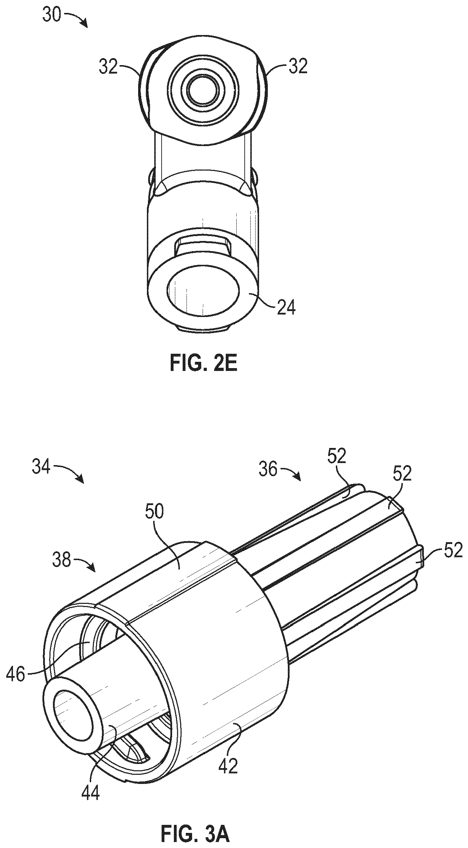

Referring now to FIG. 2A-2E, an example Y-adapter 30 of the catheter system 10 is illustrated, according to some embodiments. In some embodiments, any adapter used to connect two or more vascular access devices may be used in place of the Y-adapter 30. As illustrated in Figures 2A-2E, in some embodiments, an outer surface of the Y-adapter 30 may include one or more threads 32. In some embodiments, a second port of the Y-adapter 30 may include the outer surface having the threads 32. Thus, in some embodiments, both the Luer access port 24 and the second port of the Y-adapter may be threaded.

Referring now to FIG. 3A-3C, the catheter system 10 may include a flow control plug 34, which may be configured to couple to the Y-adapter 30, for example. In some embodiments, the flow control plug 34 may be removably coupled to the Y-adapter 30. In some embodiments, the flow control plug 34 may include a proximal end 36 and a distal end 38. In some embodiments, the proximal end 36 of the flow control plug, which may include a venting or filter element 40. In some embodiments, the filter element 40 may be gas permeable. In some embodiments, the filter element 40 may be permeable to air and not permeable to blood. In some embodiments, the distal end 38 may include a luer-lock portion, which may include a cylinder 42, although the luer-lock portion may also be generally cylindrical in some embodiments. In some embodiments, the distal end 38 may include the cylinder 42 and a taper-shaped luer tip 44 spaced apart from the cylinder 42.

In some embodiments, the cylinder 42 and the luer tip 44 may be sized and configured to connect to a female luer fitting. In some embodiments, the luer tip 44 may extend distal to a distal end of the cylinder 42. In some embodiments, an inner surface of the cylinder 42 may include one or more corresponding threads 46, which may be mated with the threads 32 to secure the flow control plug 34 to the Y-adapter 30. In some embodiments, the distal end 38 may include a stepped surface 48. In some embodiments, the distal end 38 may extend outwardly from the proximal end 36 to form the stepped surface 48. In some embodiments, the stepped surface 48 may be annular.

In some embodiments, an outer surface of the cylinder 42 may include one or more first grip elements 50. In some embodiments, the first grip elements 50 may include ribs, which may be oriented longitudinally with respect to the flow control plug 34, as illustrated in FIGS. 3A-3B. In some embodiments, the first grip elements 50 may extend from the stepped surface 48. In some embodiments, the first grip elements 50 may extend along an entire length of the cylinder 42, as illustrated, for example, in FIGS. 3A-3B, which may facilitate gripping of the distal end 38 by the user. In some embodiments, the first grip elements 50 may extend along a portion of a length of the cylinder 42.

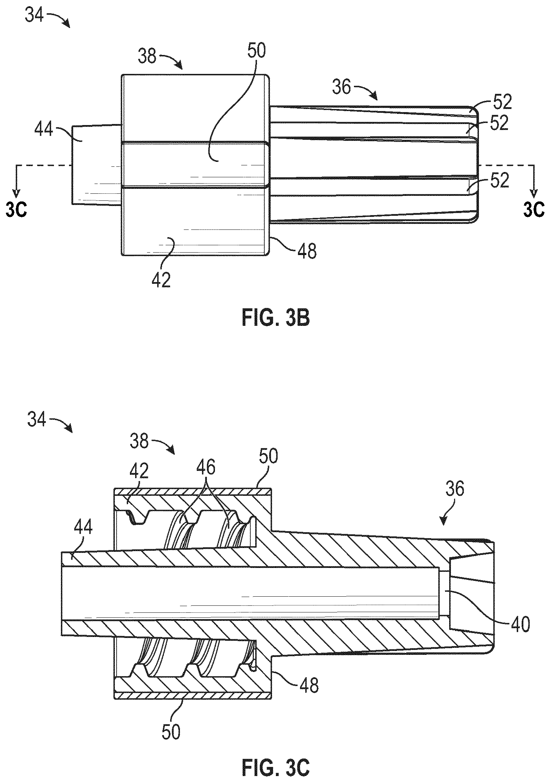

In some embodiments, an outer surface of the proximal end 36 may include one or more second grip elements 52. In some embodiments, the second grip elements 52 may include ribs, which may be oriented longitudinally with respect to the flow control plug 34. In some embodiments, the cylinder 42 may include a first number of the first grip elements 50 and/or the outer surface of the proximal end 36 may include a second number of the second grip elements 52. In some embodiments, the second number may be greater than the first number. In some embodiments, having a greater number of second grip elements 52 than first grip elements 50 may facilitate placement of a palm of a hand of the user around the proximal end 36 while a thumb of the hand is placed on the distal end 38 during coupling and/or uncoupling of the Y-adapter 30 and the flow control plug 34. In some embodiments, the proximal end 36 may include 6 to 8 second grip elements 52, which may be spaced apart around a circumference of the proximal end 36. In some embodiments, a width of each of the first grip elements 50 may be greater than a width of each of the second grip elements 52, which may facilitate gripping by the thumb. In some embodiments, the first grip elements 50 may oppose each other, as illustrated, for example, in FIG. 3C, which may facilitate gripping of the distal end 38 by the thumb of the user.

In some embodiments, the filter element 40 may include a filter paper. In some embodiments, the filter element 40 may include an acrylic copolymer membrane cast on a nonwoven nylon support. In these and other embodiments, the filter element 40 may include a VERSAPOR^{™} 800R membrane or another suitable membrane. In some embodiments, the filter element 40 may be oleophobic and/or hydrophobic material. In some embodiments, the filter element may not leak water when subjected to 5 psi minimum water pressure for 10 seconds. In some embodiments, a rate of air flow through the filter element 40 may be between 5 cc/min and 50 cc/min when subjected to 0.1 psi or 1.0 psi air pressure. In some embodiments, the filter element 40 may be attached to the proximal end of the flow control plug 34.

In some embodiments, the flow control plug 34 may be sterilized and coupled to the Y-adapter 30. The catheter system 10, including the flow control plug 34 coupled to the Y-adapter 30, may then be packaged and shipped to a destination, such as, for example, a hospital, clinic, or other facility. In some embodiments, securement of the flow control plug 34 to the Y-adapter 30 as outlined in the present disclosure may prevent the flow control plug 34 from loosening and/or falling off the Y-adapter 30 during shipment, which might otherwise occur due to due to aging or unsecure attachment of the flow control plug 34 to the Y-adapter 30. In some embodiments, securement of the flow control plug 34 to the Y-adapter 30 as outlined in the present disclosure may avoid premature uncoupling of the flow control plug 34 from the Y-adapter 30 and subsequent contamination of the flow control plug, making the flow control plug unable to be used.

In some embodiments, the flow control plug 34 may be constructed of one or more plastic materials such as, for example, polycarbonate, polypropylene, polyethylene, glycol-modified polyethylene terephthalate, acrylonitrile butadiene styrene, or any other moldable plastic material used in medical devices. In some embodiments, the flow control plug 34 may have a melting temperature of 130°C. In some embodiments, the flow control plug 34 may be compliant with ISO 594-1.

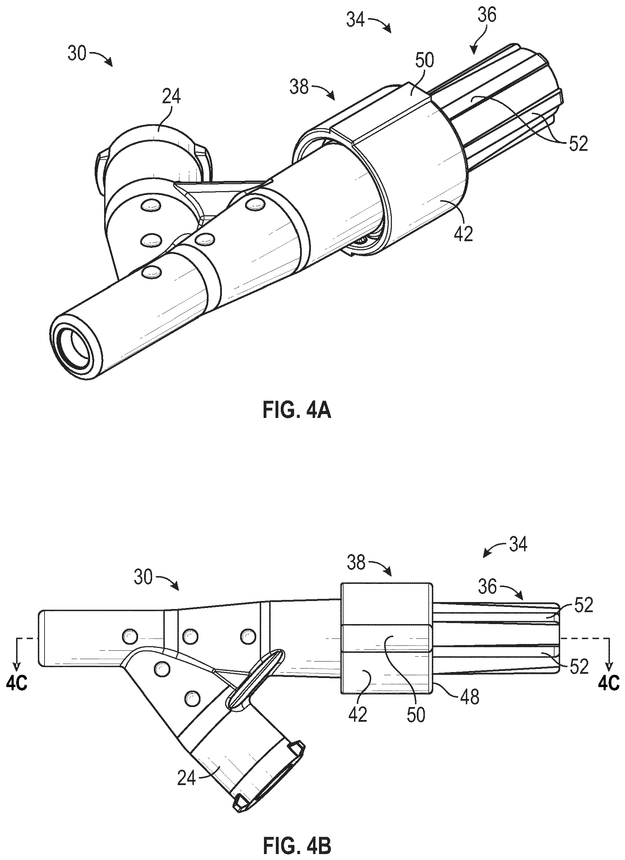

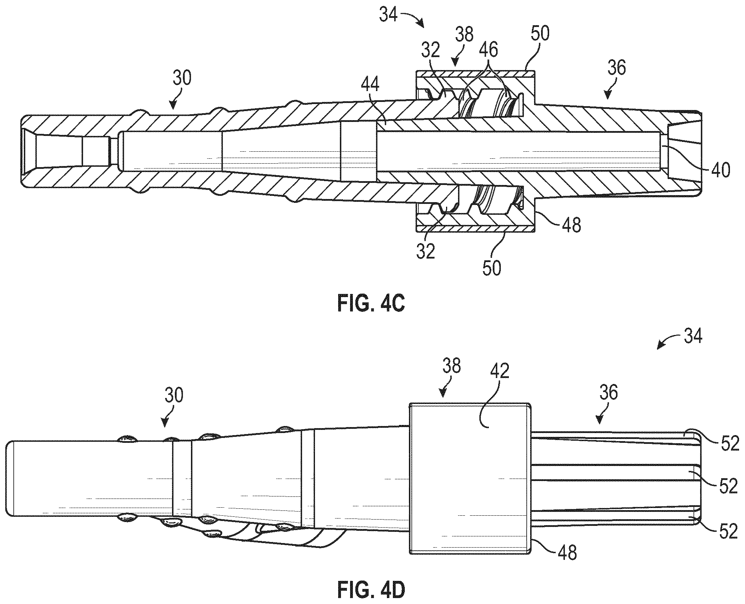

Referring now to FIGS. 4A-4D, in some embodiments, the threads 32 of the Y-adapter 30 may be mated with the corresponding threads 46 of the flow control plug 34 to secure the flow control plug 34 to the Y-adapter 30. In some embodiments, a maximum outer diameter of the distal end 38 or cylinder 42 may be greater than a maximum diameter of the proximal end 36 (which may include the second grip elements 52 protruding from a surface of the proximal end), which may facilitate gripping of the flow control plug 34 by the user. In some embodiments, the proximal end 36 may be generally cylindrical. In some embodiments, the proximal end 36 may be tapered, as illustrated, for example, in FIG. 4C.

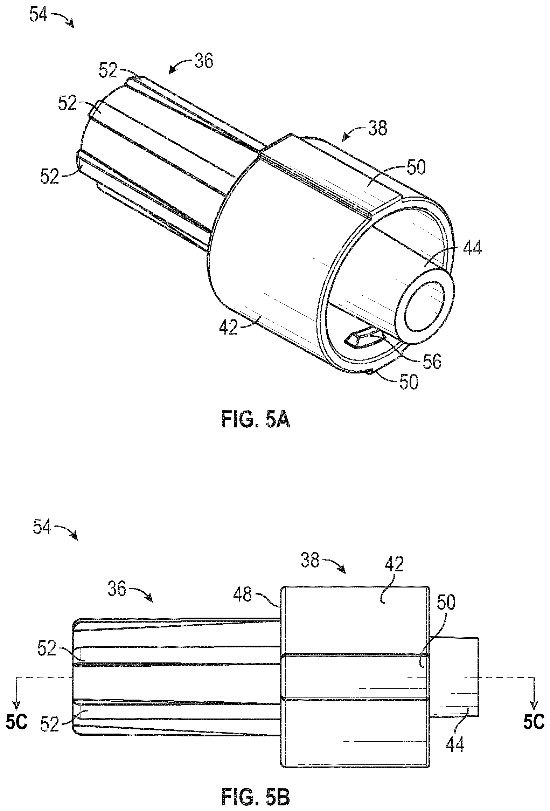

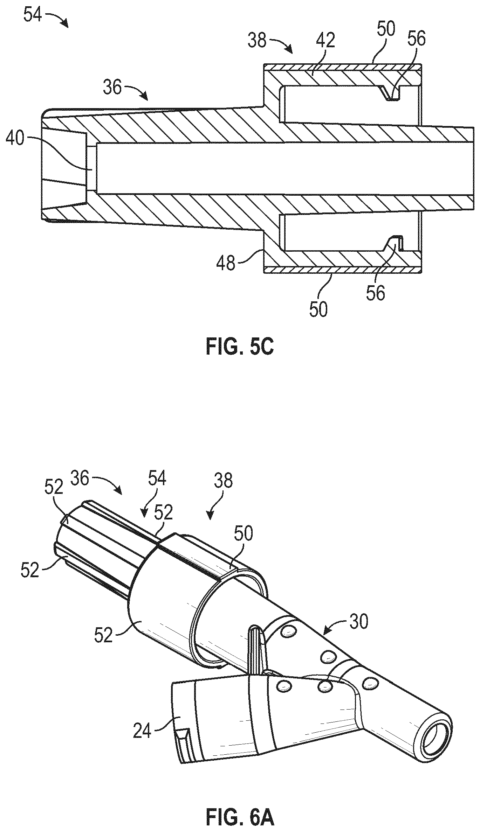

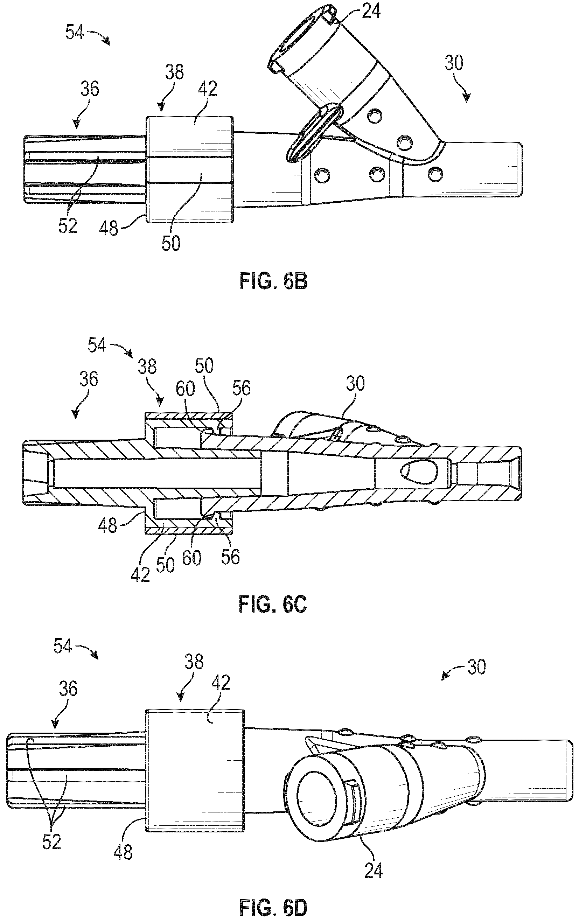

Referring now to FIGS. 5A-5C, in some embodiments, the cylinder 42 of another flow control plug 54 may include an inner surface having one or more protrusions 56, which may engage in a snap-fit with another Y-adapter, such as, for example, the Y-adapter 54 illustrated in FIGS. 6A-6C. The protrusions 56 may include various shapes and sizes. In some embodiments, the inner surface of the cylinder 42 may include one or more annular protrusions 56. In some embodiments, the protrusions 56 may include ribs or flanges. In some embodiments, the flow control plug 54 may include or correspond to the flow control plug 34 of FIGS. 3-4. In further detail, in some embodiments, the flow control plug 54 may include one or more features of the flow control plug 34. For example, the flow control plug 54 may include the first grip elements 50 and/or the second grip elements 52. As another example, the proximal end 36 of the flow control plug 54 may include the filter element 40.

Referring now to FIGS. 6A-6D, in some embodiments, in some embodiments, the protrusions 56 may engage in a snap-fit with one or more recesses 60 of a Y-adapter 58, securing the flow control plug 54 to the Y-adapter 58. In some embodiments, the recesses 60 may include various shapes and sizes configured to receive the protrusions 56 in a snap-fit. In some embodiments, the recesses 60 may each include a groove. In some embodiments, a particular recess 60 may be formed by a ridge or flange and may receive one or more particular protrusions 56 in a snap-fit. In some embodiments, the ridge may be annular. In some embodiments, the flow control plug 34 may be removably coupled to the Y-adapter 58 such that the snap-fit may be undone.

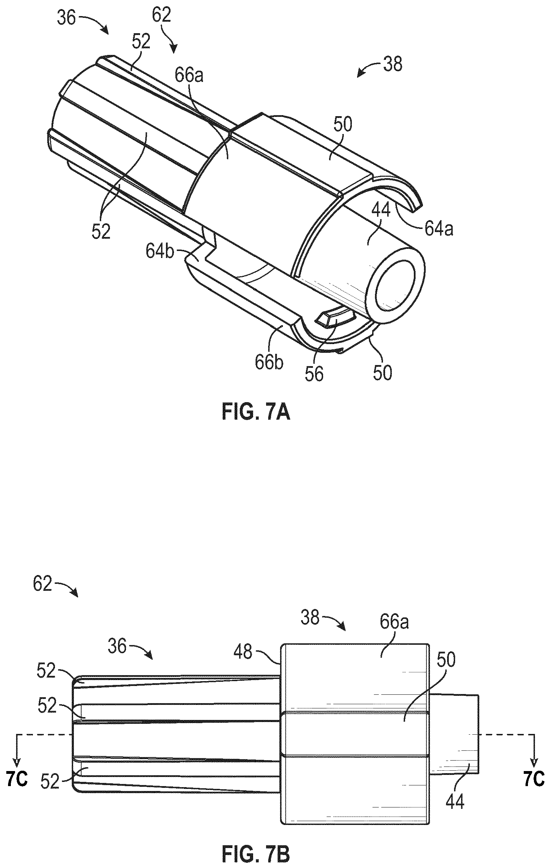

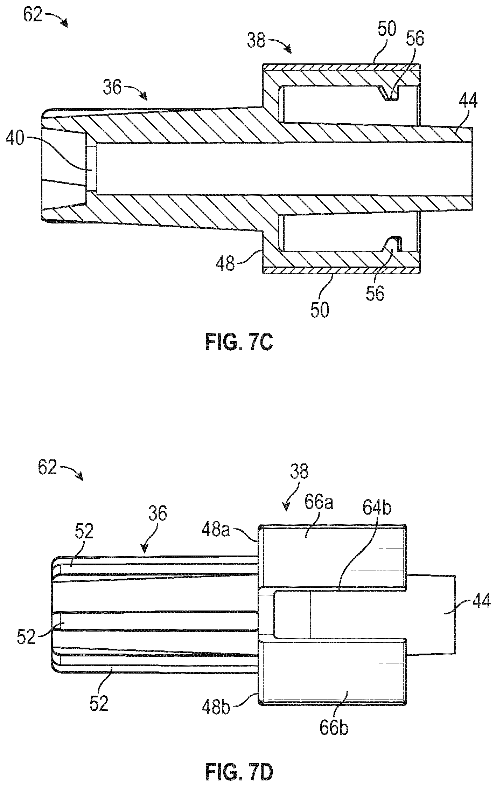

Referring now to FIGS. 7A-7D, in some embodiments, the cylinder 42 of another flow control plug 62 may include one or more slots 64, which may form multiple arms 66 of the cylinder 42. The arms 66 may be disposed between the slots 64. In some embodiments, the slots 64 may extend at least partially through the cylinder 42. In some embodiments, the slots 64 may be generally aligned with a longitudinal axis of the flow control plug 62. In some embodiments, each of the arms 66 may form a curved portion of a wall of the cylinder 42. In some embodiments, the stepped surface 48 may be divided into multiple sections by the slots 66.

In some embodiments, the slots 64 and the arms 66 may facilitate engagement of the protrusions 56 and the recesses 60 in a snap-fit as the arms 66 bias outwardly when the flow control plug is moved towards the Y-adapter, allowing the protrusions 56 to more easily access the recesses 60. In some embodiments, the slots 64 may not extend through the stepped surface 48. In some embodiments, the slots 64 may extend through the stepped surface 48 and be proximate the proximal end 36, which may allow the arms 66 to bias outwardly more easily when the flow control plug 62 is being coupled to the Y-adapter 58. In some embodiments, the slots 64 may reduce a force necessary to engage the protrusions 56 and the recesses 60 in a snap-fit. In some embodiments, ends of the protrusion 56 may be spaced apart from edges of the particular arm 66 on which the protrusion 56 is disposed, giving the protrusion 56 a length that may reduce a force necessary to remove the flow control plug 54 from the Y-adapter 54 and/or couple the flow control plug 54 to the Y-adapter 54.

In some embodiments, the flow control plug 62 may include or correspond to the flow control plug 34 of FIGS. 3-4 and/or the flow control plug 54 of FIGS. 5-6. In further detail, in some embodiments, the flow control plug 62 may include one or more features of the flow control plug 54 and/or the flow control plug 34. For example, the flow control plug 62 may include the first grip elements 50 and/or the second grip elements 52. As another example, the proximal end 36 of the flow control plug 62 may include the filter element 40. Also, the flow control plug 34 and/or the flow control plug 54 may include one or more features of the flow control plug 62.

In some embodiments, an inner surface of one or more of the arms 66 may include a particular protrusion 56, which may engage in a snap-fit with a particular recess 60. In some embodiments, the distal end 38 may include a first slot 64a and a second slot 64b (which may be referred to herein collectively as "slots 64"), and the arms 66 may include a first arm 66a and a second arm 66b (which may be referred to herein collectively as "arms 66"), as illustrated, for example, in FIG. 7A. In some embodiments, the first slot 64a and the second slot 64b may be disposed between the first arm 66a and the second arm 66b. In some embodiments, the stepped surface 66a may be proximate the first arm 66a and/or the stepped surface 66b may be proximate the second arm 66a, as illustrated, for example, in FIG. 7A.

In some embodiments, an inner surface of the first arm 66a may include a first protrusion 56. In some embodiments, an inner surface of the second arm 66b may include a second protrusion 56. In some embodiments, the first protrusion 56 may oppose the second protrusion 56. In some embodiments, the first and second protrusions 56 may be engaged in a snap-fit with a single recess 60 or with multiple recesses 60 disposed on the outer surface of the Y-adapter 58.

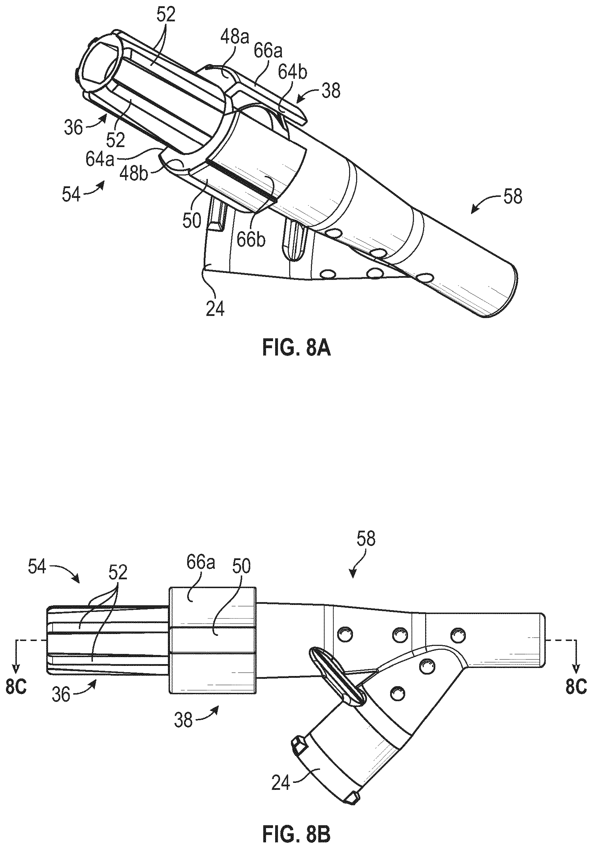

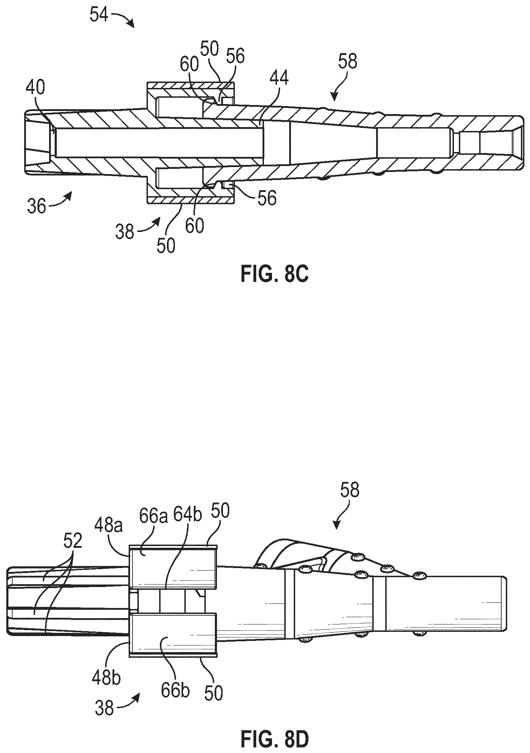

Referring now to FIGS. 8A-8D, in some embodiments, the protrusions 56 may engage in a snap-fit with one or more recesses 60 of the Y-adapter 58, securing the flow control plug 54 to the Y-adapter 58. In some embodiments, any adapter may be used to connect two or more vascular access devices may be used in place of the Y-adapter 58. It is understood that in some embodiments, the inner surface of the cylinder 42 or the inner surface of the arms 66 may include one or more recesses 60, while the outer surface of the Y-adapter 58 may include one or more protrusions 56.

In some embodiments, the flow control plug 54 and/or the flow control plug 62 may be sterilized and coupled to the Y-adapter 58. The catheter system 10, including the flow control plug 54 or the flow control plug 62 coupled to the Y-adapter 58, may then be packaged and shipped to a destination, such as, for example, a hospital, clinic, or other facility. In some embodiments, securement of the flow control plug 54 and/or the flow control plug 62 to the Y-adapter 58 as outlined in the present disclosure may prevent the flow control plug 54 and/or the flow control plug 62 from loosening and/or falling off the Y-adapter 58 during shipment, which might otherwise occur due to due to aging or unsecure attachment of the flow control plug 54 and/or the flow control plug 62 to the Y-adapter 58.

In some embodiments, a method of securing a flow control plug to a catheter system for shipment may include providing the catheter system. In some embodiments, the method may include sterilizing and/or coupling the flow control plug to a luer adapter of the catheter system before shipping the catheter system to a destination. In some embodiments, coupling the flow control plug to the luer adapter of the catheter system before shipping the catheter system to the destination includes engaging the recess with the protrusion in a snap-fit or mating the threading to the corresponding threading.

The present invention may be embodied in other specific forms without departing from its structures, methods, or other essential characteristics as broadly described herein and aspected hereinafter. The described embodiments and examples are to be considered in all respects only as illustrative, and not restrictive. The scope of the invention is, therefore, indicated by the appended aspects, rather than by the foregoing description. All changes that come within the meaning and range of equivalency of the aspects are to be embraced within their scope.

All examples and conditional language recited herein are intended for pedagogical objects to aid the reader in understanding the invention and the concepts contributed by the inventor to furthering the art, and are to be construed as being without limitation to such specifically recited examples and conditions. Although implementations of the present inventions have been described in detail, it should be understood that the various changes, substitutions, and alterations could be made hereto without departing from the scope of the invention

## Claims

1. A method of securing a flow control plug (62) to a catheter system (10) for shipment:
providing a catheter system (10), wherein the catheter system (10) comprises:
a catheter adapter (28) and a luer adapter (58) coupled to the catheter adapter (28) via extension tubing (16), wherein the luer adapter (58) includes an outer surface having a first threading or a recess (60); and
coupling a flow control plug (62) to the luer adapter (58) of the catheter system (10) before shipping the catheter system (10) to a destination, wherein the flow control plug (62) comprises:
a proximal end (36) having a filter element (40) permeable to air and not to blood; and
a distal end (38) having a cylinder (42) and a taper-shaped luer tip (44) spaced apart from the cylinder (42) and defining a distal opening, and comprising an unobstructed lumen extending between the filter element and the distal opening,
**CHARACTERIZED IN THAT**
an inner surface of the cylinder (42) includes a second threading (46) corresponding to the first threading or a protrusion (56),
wherein
coupling the flow control plug (62) to the luer adapter (58) of the catheter system (10) before shipping the catheter system (10) to the destination comprises engaging the recess (60) with the protrusion (56) in a snap-fit, or mating the first threading to the second threading corresponding to the first threading; and
wherein the cylinder comprises a plurality of slots (64) forming a plurality of arms (66) disposed between the plurality of slots (64), wherein an arm of the plurality of arms is formed between adjacent slots of the plurality of slots, wherein an inner surface of an arm of the plurality of arms (66) includes the protrusion (56), and ends of the protrusion are spaced apart from edges of the arm.

2. The method of claim 1, wherein the plurality of slots (64) extends through the cylinder (42).

3. The method of claim 1, wherein the plurality of slots (64) includes a first slot (64a) and a second slot (64b), wherein the plurality of arms (66) includes a first arm (66a) and a second arm (66b), wherein the first slot and the second slot are each disposed between the first arm and the second arm, wherein an inner surface of the first arm includes the protrusion, wherein an inner surface of the second arm includes another protrusion (56), wherein the other protrusion is engaged in a snap-fit with the recess or with another recess of the outer surface of the luer adapter (58).

4. The method of claim 1, wherein an outer surface of at least one of the plurality of arms (66) includes a grip element (50), and an outer surface of the proximal end (36) includes a plurality of grip elements (52).

5. The method of claim 4, wherein the grip element or the plurality of grip elements includes a rib oriented longitudinally with respect to the flow control plug.

6. The method of claim 1, wherein the distal end further comprises a stepped surface (48), wherein the distal end (38) extends outwardly from the proximal end (36) to form the stepped surface (48).

7. The method of claim 6, wherein the cylinder (42) comprises a plurality of arms (66) and a plurality of slots (64) disposed between the plurality of arms, wherein the plurality of slots (64) extend through the stepped surface (48).

8. The method of claim 1, wherein the luer adapter (58) is a Y-adapter.

9. A catheter system (10), comprising:
a luer adapter (58), comprising an outer surface having a recess (60), wherein the luer adapter is coupled to a catheter adapter of the catheter system via extension tubing (16);
a flow control plug (62), comprising:
a proximal end (36) having a filter element (40) permeable to air and not to blood; and
a distal end (38) having a cylinder (42) and a taper-shaped luer tip (44) spaced apart from the cylinder (42),
**CHARACTERIZED IN THAT**
an inner surface of the cylinder (42) includes a protrusion (56) engaged in a snap-fit with the recess (60); and
wherein the cylinder comprises a plurality of slots (64) forming a plurality of arms (66) disposed between the slots, wherein an arm of the plurality of arms is formed between adjacent slots of the plurality of slots, wherein an inner surface of an arm of the plurality of arms includes the protrusion, and ends of the protrusion are spaced apart from edges of the arm.

10. The catheter system of claim 9, wherein the plurality of slots extends through the cylinder.

11. The catheter system of claim 9, wherein an outer surface of at least one of the plurality of arms includes a grip element (50).

12. The catheter system of claim 11, wherein the grip element includes a rib oriented longitudinally with respect to the flow control plug.

13. The catheter system of claim 9, wherein the plurality of slots includes a first slot (64a) and a second slot (64b), wherein the plurality of arms includes a first arm (66a) and a second arm (66b), wherein the first slot and the second slot are each disposed between the first arm and the second arm, wherein an inner surface of the first arm includes the protrusion, wherein an inner surface of the second arm includes another protrusion (56), wherein the other protrusion is engaged in a snap-fit with the recess or with the outer surface of the luer adapter.

14. The catheter system of claim 9, wherein the distal end further comprises a stepped surface (48), wherein the distal end extends outwardly from the proximal end to form the stepped surface.

15. The catheter system of claim 9, wherein an outer surface of the distal end comprises a first grip element (50) comprising a plurality of first ribs, and an outer surface of the proximal end comprises a second grip element (52) comprising a plurality of second ribs, wherein the plurality of first ribs and the plurality of second ribs are oriented longitudinally with respect to the flow control plug, wherein the outer surface of the proximal end has more second ribs than the outer surface of the distal end has first ribs.

16. The catheter system of claim 15, wherein the plurality of slots (64) extends through the cylinder (42).

17. The catheter system of claim 15, wherein the plurality of slots includes a first slot (64a) and a second slot (64b), wherein the plurality of arms includes a first arm (66a) and a second arm (66b), wherein the first slot and the second slot are each disposed between the first arm and the second arm, wherein an inner surface of the first arm includes the protrusion, wherein an inner surface of the second arm includes another protrusion (56), wherein the other protrusion is configured to engage in a snap-fit with the luer adapter of the catheter system.

18. The catheter system of claim 15, wherein the distal end further comprises a stepped surface (48), wherein the distal end extends outwardly from the proximal end to form the stepped surface, wherein the stepped surface is annular.

19. The catheter system of claim 18, wherein the plurality of first ribs extends from the stepped surface and along an entire length of the cylinder.

## Patentansprüche

1. Verfahren zum Befestigen eines Durchflussregelstopfens (62) an einem Kathetersystem (10) für den Versand, umfassend:
Bereitstellen eines Kathetersystems (10), wobei das Kathetersystem (10) umfasst:
einen Katheteradapter (28) und einen Luer-Adapter (58), der über einen Verlängerungsschlauch (16) mit dem Katheteradapter (28) gekoppelt ist, wobei der Luer-Adapter (58) eine Außenfläche mit einem ersten Gewinde oder einer Aussparung (60) aufweist; und
Koppeln eines Durchflussregelstopfens (62) mit dem Luer-Adapter (58) des Kathetersystems (10) vor dem Versand des Kathetersystems (10) an einen Bestimmungsort, wobei der Durchflussregelstopfen (62) umfasst:
ein proximales Ende (36) mit einem Filterelement (40), das für Luft, jedoch nicht für Blut durchlässig ist; und
ein distales Ende (38) mit einem Zylinder (42) und einer konisch geformten Luer-Spitze (44), die von dem Zylinder (42) beabstandet ist und eine distale Öffnung definiert, und mit einem unversperrten Lumen, das sich zwischen dem Filterelement und der distalen Öffnung erstreckt,
**dadurch gekennzeichnet, dass**
eine Innenfläche des Zylinders (42) ein dem ersten Gewinde entsprechendes zweites Gewinde (46) oder einen Vorsprung (56) aufweist,
wobei das Koppeln des Durchflussregelstopfens (62) mit dem Luer-Adapter (58) des Kathetersystems (10) vor dem Versand des Kathetersystems (10) an den Bestimmungsort das Ineingriffbringen der Aussparung (60) mit dem Vorsprung (56) mittels einer Schnappverbindung oder das Ineingriffbringen des ersten Gewindes mit dem dem ersten Gewinde entsprechenden zweiten Gewinde umfasst; und
wobei der Zylinder eine Mehrzahl von Schlitzen (64) umfasst, die eine zwischen der Mehrzahl von Schlitzen (64) angeordnete Mehrzahl von Armen (66) bilden, wobei ein Arm der Mehrzahl von Armen zwischen benachbarten Schlitzen der Mehrzahl von Schlitzen ausgebildet ist,
wobei eine Innenfläche eines Arms der Mehrzahl von Armen (66) den Vorsprung (56) aufweist und Enden des Vorsprungs von Rändern des Arms beabstandet sind.

2. Verfahren nach Anspruch 1, wobei sich die Mehrzahl von Schlitzen (64) durch den Zylinder (42) hindurch erstreckt.

3. Verfahren nach Anspruch 1, wobei die Mehrzahl von Schlitzen (64) einen ersten Schlitz (64a) und einen zweiten Schlitz (64b) umfasst, wobei die Mehrzahl von Armen (66) einen ersten Arm (66a) und einen zweiten Arm (66b) umfasst, wobei der erste Schlitz und der zweite Schlitz jeweils zwischen dem ersten Arm und dem zweiten Arm angeordnet sind, wobei eine Innenfläche des ersten Arms den Vorsprung aufweist, wobei eine Innenfläche des zweiten Arms einen weiteren Vorsprung (56) aufweist und wobei der weitere Vorsprung mittels einer Schnappverbindung mit der Aussparung oder mit einer weiteren Aussparung der Außenfläche des Luer-Adapters (58) in Eingriff gebracht wird.

4. Verfahren nach Anspruch 1, wobei eine Außenfläche mindestens eines Arms der Mehrzahl von Armen (66) ein Griffelement (50) aufweist und eine Außenfläche des proximalen Endes (36) eine Mehrzahl von Griffelementen (52) aufweist.

5. Verfahren nach Anspruch 4, wobei das Griffelement oder die Mehrzahl von Griffelementen eine Rippe aufweist, die in Längsrichtung in Bezug auf den Durchflussregelstopfen ausgerichtet ist.

6. Verfahren nach Anspruch 1, wobei das distale Ende ferner eine abgestufte Fläche (48) umfasst, wobei sich das distale Ende (38) von dem proximalen Ende (36) nach außen erstreckt, um die abgestufte Fläche (48) zu bilden.

7. Verfahren nach Anspruch 6, wobei der Zylinder (42) eine Mehrzahl von Armen (66) und eine zwischen der Mehrzahl von Armen angeordnete Mehrzahl von Schlitzen (64) umfasst, wobei sich die Mehrzahl von Schlitzen (64) durch die abgestufte Fläche (48) hindurch erstreckt.

8. Verfahren nach Anspruch 1, wobei der Luer-Adapter (58) ein Y-Adapter ist.

9. Kathetersystem (10), umfassend:
einen Luer-Adapter (58), der eine Außenfläche mit einer Aussparung (60) aufweist, wobei der Luer-Adapter über einen Verlängerungsschlauch (16) mit einem Katheteradapter des Kathetersystems gekoppelt ist;
einen Durchflussregelstopfen (62), umfassend:
ein proximales Ende (36) mit einem Filterelement (40), das für Luft, jedoch nicht für Blut durchlässig ist; und
ein distales Ende (38) mit einem Zylinder (42) und einer konisch geformten Luer-Spitze (44), die von dem Zylinder (42) beabstandet ist, **dadurch gekennzeichnet, dass**
eine Innenfläche des Zylinders (42) einen Vorsprung (56) aufweist, der mittels einer Schnappverbindung mit der Aussparung (60) in Eingriff steht; und
wobei der Zylinder eine Mehrzahl von Schlitzen (64) umfasst, die eine zwischen den Schlitzen angeordnete Mehrzahl von Armen (66) bilden, wobei ein Arm der Mehrzahl von Armen zwischen benachbarten Schlitzen der Mehrzahl von Schlitzen ausgebildet ist,
wobei eine Innenfläche eines Arms der Mehrzahl von Armen den Vorsprung aufweist und Enden des Vorsprungs von Rändern des Arms beabstandet sind.

10. Kathetersystem nach Anspruch 9, wobei sich die Mehrzahl von Schlitzen durch den Zylinder hindurch erstreckt.

11. Kathetersystem nach Anspruch 9, wobei eine Außenfläche mindestens eines Arms der Mehrzahl von Armen ein Griffelement (50) aufweist.

12. Kathetersystem nach Anspruch 11, wobei das Griffelement eine Rippe aufweist, die in Längsrichtung in Bezug auf den Durchflussregelstopfen ausgerichtet ist.

13. Kathetersystem nach Anspruch 9, wobei die Mehrzahl von Schlitzen einen ersten Schlitz (64a) und einen zweiten Schlitz (64b) umfasst, wobei die Mehrzahl von Armen einen ersten Arm (66a) und einen zweiten Arm (66b) umfasst, wobei der erste Schlitz und der zweite Schlitz jeweils zwischen dem ersten Arm und dem zweiten Arm angeordnet sind, wobei eine Innenfläche des ersten Arms den Vorsprung aufweist, wobei eine Innenfläche des zweiten Arms einen weiteren Vorsprung (56) aufweist und wobei der weitere Vorsprung mittels einer Schnappverbindung mit der Aussparung oder mit der Außenfläche des Luer-Adapters in Eingriff steht.

14. Kathetersystem nach Anspruch 9, wobei das distale Ende ferner eine abgestufte Fläche (48) umfasst, wobei sich das distale Ende von dem proximalen Ende nach außen erstreckt, um die abgestufte Fläche zu bilden.

15. Kathetersystem nach Anspruch 9, wobei eine Außenfläche des distalen Endes ein erstes Griffelement (50) aufweist, das eine Mehrzahl von ersten Rippen umfasst, und eine Außenfläche des proximalen Endes ein zweites Griffelement (52) aufweist, das eine Mehrzahl von zweiten Rippen umfasst, wobei die Mehrzahl von ersten Rippen und die Mehrzahl von zweiten Rippen in Längsrichtung in Bezug auf den Durchflussregelstopfen ausgerichtet sind und wobei die Außenfläche des proximalen Endes mehr zweite Rippen aufweist als die Außenfläche des distalen Endes erste Rippen aufweist.

16. Kathetersystem nach Anspruch 15, wobei sich die Mehrzahl von Schlitzen (64) durch den Zylinder (42) hindurch erstreckt.

17. Kathetersystem nach Anspruch 15, wobei die Mehrzahl von Schlitzen einen ersten Schlitz (64a) und einen zweiten Schlitz (64b) umfasst, wobei die Mehrzahl von Armen einen ersten Arm (66a) und einen zweiten Arm (66b) umfasst, wobei der erste Schlitz und der zweite Schlitz jeweils zwischen dem ersten Arm und dem zweiten Arm angeordnet sind, wobei eine Innenfläche des ersten Arms den Vorsprung aufweist, wobei eine Innenfläche des zweiten Arms einen weiteren Vorsprung (56) aufweist und wobei der weitere Vorsprung dazu ausgebildet ist, mittels einer Schnappverbindung mit dem Luer-Adapter des Kathetersystems in Eingriff zu gelangen.

18. Kathetersystem nach Anspruch 15, wobei das distale Ende ferner eine abgestufte Fläche (48) umfasst, wobei sich das distale Ende von dem proximalen Ende nach außen erstreckt, um die abgestufte Fläche zu bilden, wobei die abgestufte Fläche ringförmig ist.

19. Kathetersystem nach Anspruch 18, wobei sich die Mehrzahl von ersten Rippen von der abgestuften Fläche aus entlang einer gesamten Länge des Zylinders erstreckt.

## Revendications

1. Procédé de fixation d'un bouchon de régulation de débit (62) à un système de cathéter (10) pour son expédition :
la fourniture d'un système de cathéter (10), dans lequel le système de cathéter (10) comprend :
un adaptateur de cathéter (28) et un adaptateur Luer (58) couplé à l'adaptateur de cathéter (28) par l'intermédiaire d'une tubulure d'extension (16), où l'adaptateur Luer (58) comprend une surface externe ayant un premier filetage ou un évidement (60) ; et
le couplage d'un bouchon de régulation de débit (62) à l'adaptateur Luer (58) du système de cathéter (10) avant l'expédition du système de cathéter (10) vers une destination, où le bouchon de régulation de débit (62) comprend :
une extrémité proximale (36) ayant un élément filtrant (40) perméable à l'air mais pas au sang ; et
une extrémité distale (38) ayant un cylindre (42) et une pointe Luer de forme conique (44) espacée du cylindre (42) et définissant une ouverture distale, et comprenant une lumière non obstruée s'étendant entre l'élément filtrant et l'ouverture distale,
**CARACTÉRISÉ EN CE QUE**
une surface interne du cylindre (42) comprend un second filetage (46) correspondant au premier filetage ou une saillie (56),
dans lequel
le couplage du bouchon de régulation de débit (62) à l'adaptateur Luer (58) du système de cathéter (10) avant l'expédition du système de cathéter (10) vers la destination comprend l'engagement de l'évidement (60) avec la saillie (56) par encliquetage, ou l'accouplement du premier filetage au second filetage correspondant au premier filetage ; et
dans lequel le cylindre comprend une pluralité de fentes (64) formant une pluralité de bras (66) disposés entre la pluralité de fentes (64), dans lequel un bras de la pluralité de bras est formé entre des fentes adjacentes de la pluralité de fentes,
dans lequel une surface interne d'un bras de la pluralité de bras (66) comprend la saillie (56), et les extrémités de la saillie sont espacées des bords du bras.

2. Procédé selon la revendication 1, dans lequel la pluralité de fentes (64) s'étend à travers le cylindre (42).

3. Procédé selon la revendication 1, dans lequel la pluralité de fentes (64) comprend une première fente (64a) et une seconde fente (64b), dans lequel la pluralité de bras (66) comprend un premier bras (66a) et un second bras (66b), dans lequel la première fente et la seconde fente sont chacune disposées entre le premier bras et le second bras, dans lequel une surface interne du premier bras comprend la saillie, dans lequel une surface interne du second bras comprend une autre saillie (56), dans lequel l'autre saillie est engagée par encliquetage dans l'évidement ou dans un autre évidement de la surface externe de l'adaptateur Luer (58).

4. Procédé selon la revendication 1, dans lequel une surface externe d'au moins un de la pluralité de bras (66) comprend un élément de préhension (50), et une surface externe de l'extrémité proximale (36) comprend une pluralité d'éléments de préhension (52).

5. Procédé selon la revendication 4, dans lequel l'élément de préhension ou la pluralité d'éléments de préhension comprend une nervure orientée longitudinalement par rapport au bouchon de régulation de débit.

6. Procédé selon la revendication 1, dans lequel l'extrémité distale comprend en outre une surface étagée (48), dans lequel l'extrémité distale (38) s'étend vers l'extérieur à partir de l'extrémité proximale (36) pour former la surface étagée (48).

7. Procédé selon la revendication 6, dans lequel le cylindre (42) comprend une pluralité de bras (66) et une pluralité de fentes (64) disposées entre la pluralité de bras, dans lequel la pluralité de fentes (64) s'étendent à travers la surface étagée (48).

8. Procédé selon la revendication 1, dans lequel l'adaptateur Luer (58) est un adaptateur en Y.

9. Système de cathéter (10), comprenant :
un adaptateur Luer (58), comprenant une surface externe ayant un évidement (60), où l'adaptateur Luer est couplé à un adaptateur de cathéter du système de cathéter par l'intermédiaire d'une tubulure d'extension (16) ;
un bouchon de régulation de débit (62), comprenant :
une extrémité proximale (36) ayant un élément filtrant (40) perméable à l'air mais pas au sang ; et
une extrémité distale (38) ayant un cylindre (42) et une pointe Luer de forme conique (44) espacée du cylindre (42),
**CARACTÉRISÉ EN CE QUE**
une surface interne du cylindre (42) comprend une saillie (56) engagée par encliquetage avec l'évidement (60) ; et
dans lequel le cylindre comprend une pluralité de fentes (64) formant une pluralité de bras (66) disposés entre les fentes, dans lequel un bras de la pluralité de bras est formé entre des fentes adjacentes de la pluralité de fentes,
dans lequel une surface interne d'un bras de la pluralité de bras comprend la saillie, et les extrémités de la saillie sont espacées des bords du bras.

10. Système de cathéter selon la revendication 9, dans lequel la pluralité de fentes s'étend à travers le cylindre.

11. Système de cathéter selon la revendication 9, dans lequel une surface externe d'au moins un de la pluralité de bras comprend un élément de préhension (50).

12. Système de cathéter selon la revendication 11, dans lequel l'élément de préhension comprend une nervure orientée longitudinalement par rapport au bouchon de régulation de débit.

13. Système de cathéter selon la revendication 9, dans lequel la pluralité de fentes comprend une première fente (64a) et une seconde fente (64b), dans lequel la pluralité de bras comprend un premier bras (66a) et un second bras (66b), dans lequel la première fente et la seconde fente sont chacune disposées entre le premier bras et le second bras, dans lequel une surface interne du premier bras comprend la saillie, dans lequel une surface interne du second bras comprend une autre saillie (56), dans lequel l'autre saillie est engagée par encliquetage avec l'évidement ou avec la surface externe de l'adaptateur Luer.

14. Système de cathéter selon la revendication 9, dans lequel l'extrémité distale comprend en outre une surface étagée (48), dans lequel l'extrémité distale s'étend vers l'extérieur à partir de l'extrémité proximale pour former la surface étagée.

15. Système de cathéter selon la revendication 9, dans lequel une surface externe de l'extrémité distale comprend un premier élément de préhension (50) comprenant une pluralité de premières nervures, et une surface externe de l'extrémité proximale comprend un second élément de préhension (52) comprenant une pluralité de secondes nervures, dans lequel la pluralité de premières nervures et la pluralité de secondes nervures sont orientées longitudinalement par rapport au bouchon de régulation de débit, dans lequel le nombre de secondes nervures sur la surface externe de l'extrémité proximale est supérieur au nombre de premières nervures sur la surface externe de l'extrémité distale.

16. Système de cathéter selon la revendication 15, dans lequel la pluralité de fentes (64) s'étend à travers le cylindre (42).

17. Système de cathéter selon la revendication 15, dans lequel la pluralité de fentes comprend une première fente (64a) et une seconde fente (64b), dans lequel la pluralité de bras comprend un premier bras (66a) et un second bras (66b), dans lequel la première fente et la seconde fente sont chacune disposées entre le premier bras et le second bras, dans lequel une surface interne du premier bras comprend la saillie, dans lequel une surface interne du second bras comprend une autre saillie (56), dans lequel l'autre saillie est configurée pour s'engager par encliquetage avec l'adaptateur Luer du système de cathéter.

18. Système de cathéter selon la revendication 15, dans lequel l'extrémité distale comprend en outre une surface étagée (48), dans lequel l'extrémité distale s'étend vers l'extérieur à partir de l'extrémité proximale pour former la surface étagée, dans lequel la surface étagée est annulaire.

19. Système de cathéter selon la revendication 18, dans lequel la pluralité de premières nervures s'étend à partir de la surface étagée et sur toute la longueur du cylindre.
